# EUROPEAN PATENT APPLICATION

(11) **EP 0 576 302 A1**
(43) Date of publication of application: **29.12.1993**
(21) Application number: 93305000.7
(22) Date of filing: 25.06.1993
(51) Int. Cl.: A61M 5/32, A61M 25/06

(54) **Catheter with extensible, two-piece needle guard**

(30) Priority: 26.06.1992 US 904808
(71) Applicant: CRITIKON, INC., Tampa Florida 33634 (US)
(72) Inventor: Lemieux, Francis P., Palm Harbor, Florida 34683 (US); Chang, Joseph J., Tampa, Florida 33625 (US)
(74) Representative: Fisher, Adrian John

(57) **Abstract**

A catheter device is described with a safety needle guard (30) that covers and protects the needle after use of the device. The device includes a semi-tubular needle housing (20) containing a flash chamber with a hollow needle (24) extending from the distal end of the flash chamber (26). A tubular needle guard concentrically fits and slides within the needle housing. The needle guard is of two-piece construction and has a longitudinal slot (36) through which the mounting base (27) of the flash chamber passes as the guard slides within the housing. The top of the semi-tubular housing is open so that a user may access the top of the tubular needle guard with a finger to urge the needle guard to an extended position from the distal end of the housing and in a surrounding position about the needle. As the telescopic needle guard attains its fully extended position about the needle, it locks in place in the needle housing, and both pieces also work in place.

## Description

### FIELD OF THE INVENTION

This invention relates to intravascular (I.V.) catheters and, in particular, to I.V. catheter assemblies which cover the needle point after use to prevent accidental injury from used needles.

### BACKGROUND OF THE INVENTION

Intravenous catheters for the infusion of fluids into the peripheral veins of a patient are one of the most common devices used in I.V. therapy. I.V. catheters may be produced in two general forms: through-the-needle catheters, in which a catheter is threaded through the needle cannula and into the vein of a patient, and over-the-needle catheters, in which the needle and concentric outer catheter are inserted into the vein and the needle is withdrawn through the emplaced catheter.

A typical over-the-needle I.V. catheter assembly requires the user to remove and then dispose of a contaminated needle after the needle tip and catheter are properly located in a blood vessel of a patient. Once the needle is withdrawn from the catheter, the user's immediate priorities are infusion set connection and site preparation, including the taping of the catheter to the patient. Because of the urgency of these procedures, the needle is normally just dropped conveniently nearby and then retrieved later. Since the needle at this time is exposed and located close to where the user is completing work with the catheter, accidental self-inflicted needle injuries are not uncommon. For reasons of the desirability of protecting the user from exposure to blood borne disease such as hepatitis and AIDS, there is an increasing need to protect the user from accidental needle injury.

A catheter design which is directed toward this need is shown in Luther, United States Patent number 4,762,516. The catheter shown in this application includes an elongate body which houses a sliding needle guard. In use, the needle with its surrounding catheter tube is inserted through the skin of a patient until the tip of the needle is located in a blood vessel, a position detected by a small flow of blood through the needle and into the flash chamber of the catheter. The user then advances a tab on the top of the needle guard to simultaneously thread the catheter tube into the blood vessel and begin the retraction of the needle from the catheter tube. As the needle is withdrawn from the emplaced catheter, the advance of the tab slides the needle guard out of the housing and along the needle, until the distal end of the guard covers the needle tip and the proximal end of the guard locks in the elongate body. The needle and guard may then be set aside with the needle tip fully protected.

While the arrangement described in Luther '516 can provide full protection against accidental needle injury, it is sometimes desirable to provide such a catheter in a smaller, smoothly operating configuration which can be readily manipulated by small hands. In accordance with the principles of Ducharme et al., U.S. Patent No 5,000,740, a catheter assembly with needle guard is provided with a semi-tubular needle housing that is open on the upper surface. Located within the housing is a flash chamber with a needle extending from the distal end of the chamber and beyond the distal end of the housing. A tubular needle guard is located for distal movement within the semi-tubular needle housing, and has a distal opening through which the needle extends. The bottom of the needle guard is slotted to fit around the base of the flash chamber. At the rear of the needle guard slot is a portion of a locking mechanism which will engage with and lock in the needle housing when the needle guard is extended to cover the needle.

In a preferred embodiment of the Ducharme et al. '740 invention, the needle guard includes a separate tip piece which enables the mounting of a catheter hub over the needle guard tip. The use of a separate tip also facilitates automated assembly without damage to the sharp pointed needle. When the needle guard is extended, the distal end of the tip piece extends beyond the point of the needle. The needle housing of the preferred embodiment also includes an integral, contoured finger grip located on each side of the needle housing. The catheter assembly is further provided with a protective sheath to protect the catheter and needle prior to use.

Yet, a perceived need not met by Ducharme et al. '740 is to provide a compact unit comprising a catheter with a needle guard, such that the unit may fit comfortably within the hand of the user. However, in certain instances, it may still be necessary to provide a compact needle guard which is extensible to longer lengths than a "typical" needle guard. This may be true, for instance, when the user must rely on a longer length needle; or, if a side port is being used, such that more reliable flow patterns may be discerned by a longer flash chamber. Or, perhaps, conversely the user requires less space for storage, and yet still needs a typical length needle guard.

### SUMMARY OF THE INVENTION

It is an object of the invention to provide a catheter device containing a longer length needle guard.

It is further an object of the invention to provide a catheter device wherein there is a needle guard capable of collapsing into itself, such that it may be compactly stored.

It is yet another object of the invention to provide a catheter device wherein the needle guard may be used with side port catheter hook-ups, or with longer length needles.

These, and other objects of the invention are provided in a catheter assembly comprising: a catheter with a catheter hub attached thereto, a needle with a needle hub attached thereto, the needle insertable into the catheter, and a needle guard slidable over said needle to cover the tip of said needle. The needle guard is of longer length, typically greater than 1.25'', due to its capabilities of being extended from a "stored" compact position to an open, "telescoped" position. Typically, this needle guard is of two piece construction with one piece locking to the other in the extended position. The needle guard, in turn, locks over the needle, typically to a housing on which the needle is attached.

The inventions are described herein will be better understood from the attached drawings and the Detailed Description of the Invention.

### DESCRIPTION OF THE DRAWINGS:

FIGURE 1 is perspective view of a catheter assembly constructed in accordance with the principles of the prior art Ducharme invention with the sheath in place;
FIGURE 2 is a perspective view of the catheter assembly of FIGURE 1 after removal of the sheath;
FIGURE 3 is a perspective view of the needle housing, needle, and needle guard of the catheter assembly of FIGURE 2 with the catheter removed;
FIGURE 4 is a perspective view of the assembly of FIGURE 3 with the needle guard extended;
FIGURE 5 is a cross-sectional view of the catheter assembly of FIGURE 2;
FIGURE 6 is a bottom view of the first piece of the needle guard of this invention;
FIGURE 6a is a side elevation view of the element of FIGURE 6;
FIGURE 7 is a bottom view of the second, extensible piece of the needle guard of this invention;
FIGURE 7a is a side elevation view of the element of FIGURE 7;
FIGURE 8 is a bottom view of the elements of FIGURES 6 and 7 in their assembled and ready-for-use position;
FIGURE 8a is a bottom view of the elements of FIGURE 8 in their fully extended and locked positions;
FIGURE 9 is a perspective view of an alternate form of the element of FIGURE 7;
FIGURE 9a is a perspective view of an alternate form of the element of FIGURE 6, which is to be used with the element of FIGURE 9; and
FIGURE 9b is a top view of the element of FIGURE 9.

### DETAILED DESCRIPTION OF THE INVENTION

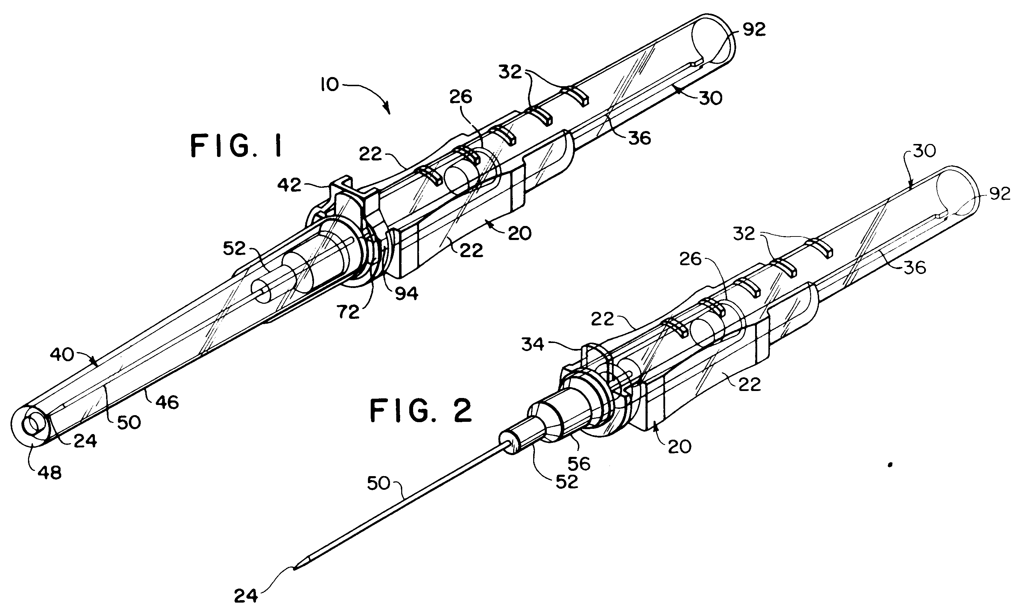

Referring first to FIGURE 1, a catheter assembly 10 constructed in accordance with the principles of the Ducharme et al. '740 invention is shown. This catheter assembly 10 is to be modified in accordance with this invention, but one must first understand the principles of the use of catheter assembly 10. The assembly 10 includes a needle housing 20 which is semi-tubular in shape and open at the top. Molded on the sides of the needle housing 20 are opposing contoured finger grips 22, one of which is visible in FIGURE 1. Located inside the semi-tubular needle housing and extending proximally therefrom is a tubular needle guard 30. On the upper surface of the needle guard are a number of small projections 32 which provide surfaces against which a user may press to fully extend the needle guard. These projections permit a user to extend the needle guard with the index or other finger while holding the catheter assembly with one hand. Extending distally from the needle housing 20 is a protective sheath 40 which covers the distally extending needle and catheter.

FIGURE 2 illustrates the assembly of FIGURE 1 after removal of the sheath 40. This drawing shows the catheter 50 and its catheter hub 52 mounted on the distal end of the needle guard 30. The point of the needle 24 is seen to extend from the distal tip of the catheter 50. A push-off tab 34 is seen projecting upward from the needle guard proximal the catheter hub 52.

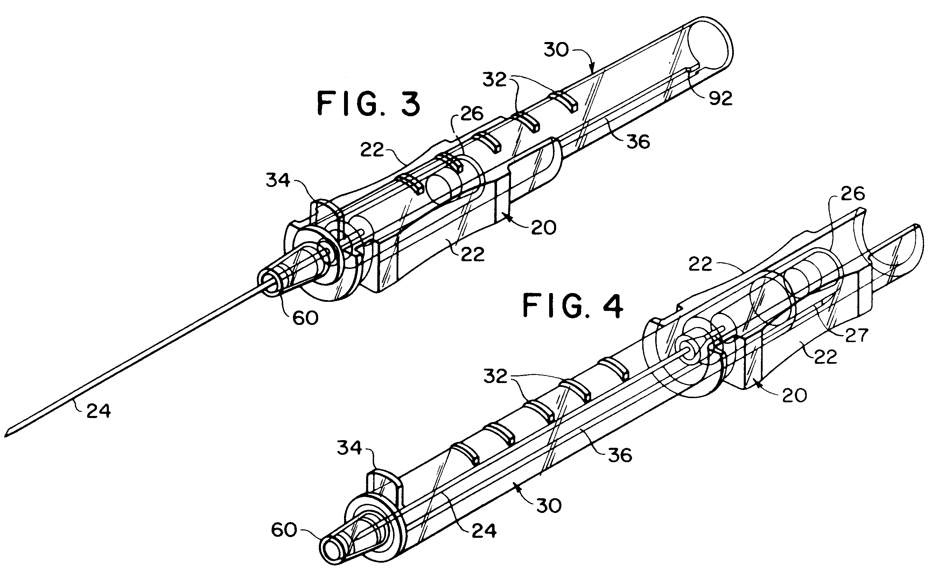

FIGURE 3 shows the assembly of FIGURE 2 prior to mounting the catheter and hub on the distal end of the needle guard. Located on the distal end of the needle guard is a needle guard tip 60, through which the needle 24 extends. FIGURE 4 shows the assembly of FIGURE 3 after the needle guard 30 has been extended to cover the needle 24. In this position the needle guard is locked in its extended position inside the needle housing, and the point of the needle is located inside of the needle guard tip 60.

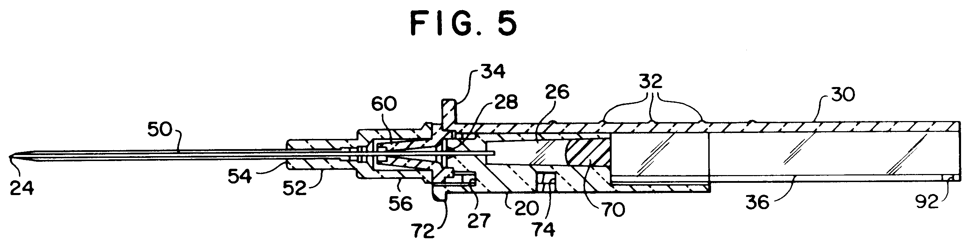

FIGURE 5 is a cross-sectional view of the catheter assembly of FIGURE 2. The catheter 50 is seen to extend from the distal end 54 of the catheter hub 52 and is concentric therewith. The catheter may be attached to its hub by any means known in the art, including adhesively or mechanically by means of a metal eyelet. The larger diameter proximal portion 56 of the catheter hub 52 is flanged at its proximal end for connection to an infusion set, and the inner diameter of the proximal portion of the hub is sized to fit over the distal portion of the needle guard tip 60.

The needle 24 is attached to the distal end of the flash chamber 26 of the needle housing with the proximal end of the needle terminating within the chamber. The needle 24 is affixed in place by adhesive 28. The needle extends through the needle guard tip 60, the needle hub 52, and the catheter 50, with the point of the needle extending from the distal end of the catheter. The rear of the flash chamber 26 is plugged by a microporous plug 70. The needle guard is seen to extend proximal the rear of the needle housing with the needle guard tip 60 affixed to the distal end of the needle guard at the location of the push-off tab 34. The tubular needle guard surrounds the flash chamber 26, with the base 27 of the flash chamber being located in a longitudinal slot 36 at the bottom of the needle guard. As the needle guard slides in the distal direction to cover the needle it is maintained concentric with the needle housing by the concentric tubular construction of the needle housing and needle guard and by the tracking of the base 27 of the flash chamber in the needle guard slot 36.

The subassembly of the needle 24, the needle housing 20, the needle guard 30, the porous flash plug 70, and the needle guard tip 60 can be seen especially well in Figure 5. Assembly may be accomplished by inserting the flash plug 70 into the proximal end of the flash chamber 26. The needle 24 is inserted into the distal end of the flash chamber and is adhesively secured in place. With the needle and housing oriented vertically, the needle guard 30 is dropped over the needle. The large internal opening of the needle guard minimized the possibility of contact between the needle guard and the point of the needle, which is important to prevent damage to the sharp needle point during assembly. The needle guard then slides into the needle housing from the distal end of the housing. The tapered distal end 84 of the flash chamber base engages the proximal end of the guard slot 36 to guide the needle guard into the housing around the base 27 of the flash chamber. The guard and housing will slide together until the narrowed proximal end 92 of the slot engages the aperture 74 of the housing, causing the two components to lock together. An instrument is inserted into the aperture 74 and into slot 36 to spread the narrowed portion 92 of the slot and thereby permit the needle guard to proceed fully into the needle housing.

However, the concentric tubular construction of the needle guard and housing also permits the needle guard to slide into the housing from the proximal end of the housing. This is preferable to the distal entry technique described above, for the catheter device can then be assembled without causing the needle guard to pass through its locking position, thereby obviating the need to unlock the narrowed portion 92 of the guard slot during assembly of the device.

With the distal end of the needle guard extending beyond the distal end of the housing, the needle guard tip 60 is dropped over the point of the needle. The small tip can be accurately aligned with its central passageway in line with the needle so that the guard tip can be slipped over the needle without damaging the point of the needle. When the proximal end 62 of the guard tip fully engages the distal opening 90 of the needle guard these two components are ultrasonically welded together. This two-component needle guard thus permits assembly of the catheter device without damage to the needle. The needle guard and tip then slide fully into the needle housing. The catheter 50 and hub 52 are then slipped over the needle 24 until the catheter hub 52 is securely seated over the tapered surface 64 of the needle guard tip, as shown in FIGURE 5. The protective sheath may then be slipped over the catheter and needle and snapped onto the needle housing flange 72. The catheter assembly is then packaged for delivery to a user.

The catheter assembly of FIGURE 5 may be used in the conventional manner by inserting the concentric catheter and needle through the skin of a patient and into a blood vessel. When the point of the needle 24 is properly located in the vessel, a small amount of blood will flow through the needle and into the flash chamber 26. Since the needle housing and guard are made of transparent or translucent polymeric materials, the flow of blood will be readily apparent in the flash chamber. The needle is then retracted from the vessel and the catheter 50 threaded into the vessel by grasping the finger grips 22 of the housing with the thumb and fingers and pushing the push-off tab 34 in the distal direction with one finger. This motion will push the catheter hub 52 off of the needle guard tip 60 to advance the catheter. As the needle guard begins to extend out from the distal end of the needle housing such that the push-off tab 34 is beyond the reach of the finger of the user, the user may engage the projections 32 with the finger to continue the distal motion of the needle guard.

Finally this motion will result in proper threading of the catheter into the vessel and the complete withdrawal of the needle from the patient's body. The needle guard 30 is then advanced to its fullest extension. As it does so, the tapered proximal section 82 of the flash chamber base will spread the narrowed proximal portion 92 of the needle guard slot 36 until the narrowed portion 92 finally engages the aperture 74. At the fullest extension of the needle guard from the housing the engagement of the narrowed portion 92 in the aperture 74 will lock the needle guard in its protective position. The needle, housing and guard may then be set aside without concern for inadvertent injury to the user or others.

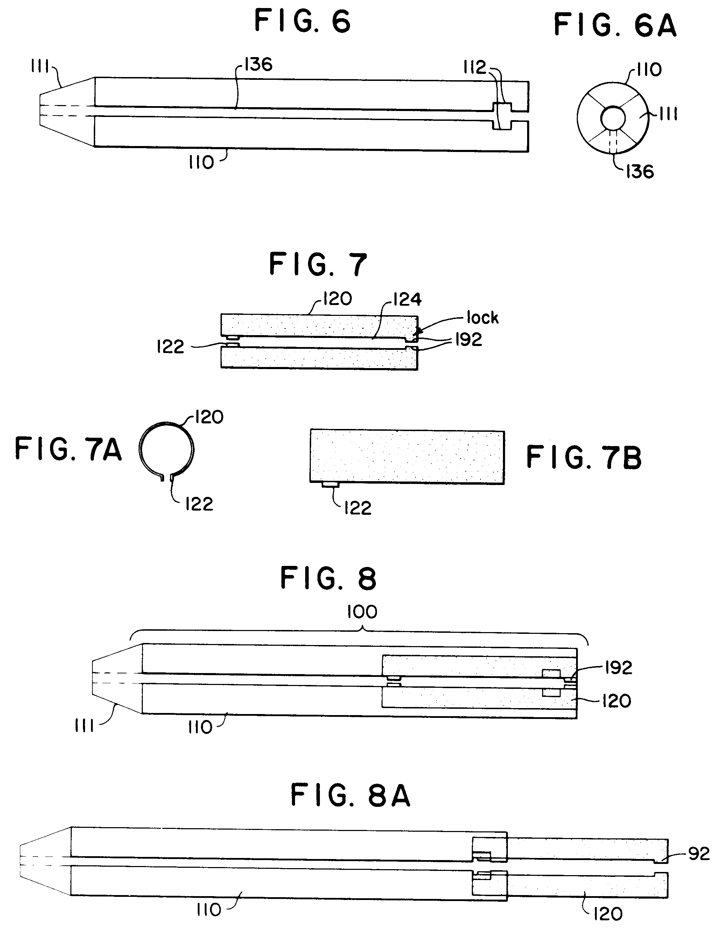

As seen in the FIGURES 6 through 9 et al., there is disclosed a modified needle guard which enables the user to extend the needle guard in a telescopic fashion. This needle guard assembly 100 is intended to be used with the catheter assembly 10 as described in FIGURES 1-5. The needle guard assembly 100 is intended to perform exactly like needle guard 30 as seen in the embodiments of FIGURES 1 through 5.

Needle guard assembly 100 is comprised of a two piece member. The first piece comprises needle guard 110. This needle guard 110 is intended to contain a similar needle guard hub 111 which will mate with the catheter hub 56, as seen in FIGURES 1 through 5. The one difference of the needle guard portion 110 is that it now contains a pair of slots 112 rather than the locking teeth 92 as seen in needle guard 30. These slots 112 are extended wider than the slot 136 which corresponds to slot 36 of the needle guard 30 of FIGURES 1 through 5. Therefore, the slots 112 on this new portion 110 are no longer capable of locking against flange 74 as described in FIGURES 1 through 5. Rather, this portion 110 of the needle guard is intended solely to cover the tip of a needle 24, and to be slidable within the needle housing 20 as described previously.

As seen in FIGURES 7, 7a and 7b, there is described an extending or second portion of the needle guard 120. This extending portion 120 has a diameter which is smaller than the diameter of the portion 110 so that it fits comfortably therein. As can be seen especially in FIGURES 7a and 7b there are contained tabs 122 which extend wider than the circumference of the second portion 120. These tabs 122 are intended to mate with the slots 112 on the first portion 110 of the mechanism. Furthermore, there is contained a slot 124 in this telescoping needle guard which also rides comfortably within the flange 74 of the needle housing. As can be further seen, there are now contained locking teeth 192 which are very similar to the teeth 92 of the earlier described invention. It is these locking teeth 192 which are intended to engage the aperture 74 of needle housing in order to lock the extended needle guard assembly 100 thereon.

As seen in FIGURE 8 and 8a, there is described the new needle guard assembly 100 of both before and during use. Initially, this needle guard 100 is contained so that the second portion 120 is held within the first portion 110. When the needle guard is extended, the tabs 122 on second portion 120 engage the slots 112 on first portion 110. Then, the needle guard takes the configuration of the element described by FIGURE 8a.

Now, in usage, the needle guard 100 of the present invention is assembled as in FIGURE 8. Second portion 120 and first portion 110 fit comfortably within the needle housing. Also, a catheter sits at the proximal end of the needle guard assembly 100. Although this is not shown in the figures, it is identical to the description as in FIGURES 1 through 5. During use, the needle guard assembly 100 is slid over the end of the needle. During this motion, as some point the locking tabs 192 on second portion 120 will engage the flange 74, much as in the description of the first embodiment. Thereafter, through continued pushing of the first portion 110 of the needle guard, the first portion 110 and second portion 120 mate so that the locking tabs 122 are now bottomed in the slots 112 created in first portion 110. Now, the needle guard assumes its final telescoped extended position.

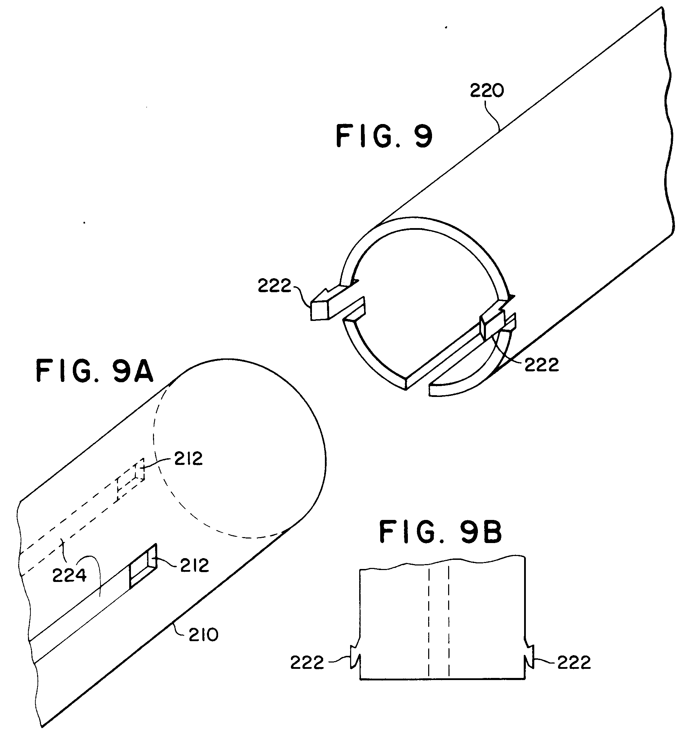

As further seen in FIGURES 9, 9a and 9b, there is described an alternate embodiment of the needle guard portions 110, 120. These can be seen in embodiment portions 210, 220. There, the extender portion 220 contains a pair of tabs 222 which extend outward of the circumference of the device at 90° angles to the slot contained in the extender. Furthermore, the first portion 210 now contains holes 212 which are created in the first portion 210 at 90° angles to a slot 214 contained in the first portion. In this way, there is more stable locking of one portion to the other. Of course, in order to make it relatively easy for motion in this direction, there may be contained channels or grooves 224 leading from the distal to the proximal portion of piece 210 to approach the locking holes 212.

Of course, it is to be understood that the foregoing invention is to be derived from the attached claims and their equivalents.

## Claims

1. A catheter mechanism having a proximal and distal end containing:
a catheter,
a needle insertable into said catheter, said needle attached to a housing; and
a needle guard slidable with respect to said housing, said needle guard movable from a non-shielded position wherein said needle is exposed to a shielded position wherein said needle is covered by said needle guard;
wherein said needle guard is of a construction such that at the proximal end of the catheter mechanism, said needle guard is able to telescope into itself.

2. The catheter mechanism of claim 1 wherein the needle guard is of two piece construction.

3. The catheter mechanism of claim 2 further containing means partially on said needle guard and partially in said housing for locking said needle guard in its shielded position.

4. The catheter mechanism of claim 2 wherein a first of said pieces is located proximal a second of said pieces, and said first piece fits within said second piece when said needle guard in not in its extended position.

5. A catheter assembly comprising:
a needle housing;
a flash chamber located in the exterior of said needle housing and having a hollow needle extending from the distal end thereof;
a tubular needle guard slideably located within said needle housing and including an aperture at its distal end for passage of said hollow needle therethrough;
a catheter and catheter hub assembly suitable for mounting on the distal end of said needle guard; and
means for locking said needle guard in an extended position relative to the distal end of said needle housing;
wherein said flash chamber is located within said slideable needle guard; and
wherein said flash chamber further includes a base for securing said flash chamber within said needle housing, and wherein said needle guard further includes a longitudinally extending slot which engages said flash chamber base as said needle guard slides within said needle housing; and
wherein said needle guard is of a two piece construction such that one said piece can telescope into the said second said piece.

6. The catheter mechanism of claim 5 wherein said needle guard has a slot in both said pieces, said needle guard slidable along said slot with respect to said base.

7. The catheter mechanism of claim 5 wherein said two pieces are capable of being locked one with respect to the other when said needle guard is fully extended.

8. The catheter mechanism of claim 7 wherein said locking mechanism for said two piece assembly is located on said slot.

9. The catheter mechanism of claim 7 wherein said locking mechanism for said two piece assembly is located at 90° angles to said slot.

10. The catheter mechanism of claim 5 wherein said needle guard is at least 1.25'' in length.

11. The catheter mechanism of claim 5 wherein a first of said pieces is located proximal a second of said pieces, and said first piece fits within said second piece when said needle guard in not in its extended position.
